# EUROPEAN PATENT SPECIFICATION

(11) **EP 1 908 420 B1**
(45) Date of publication and mention of the grant of the patent: **25.11.2009**
(21) Application number: 06021057.2
(22) Date of filing: 06.10.2006
(51) Int. Cl.: A61B 17/11

(54) **Anastomotic ring device with locking means**
Ringanastomose mit Schliessmittel
Dispositif d'anneau d'anastomose avec ferrure de verrouillage

(43) Date of publication of application: 09.04.2008
(73) Proprietor: ETHICON ENDO-SURGERY, INC., Cincinnati, OH 45242-2839 (US)
(72) Inventor: Pastorelli, Alessandro, 00136 Roma (IT); Tacchino, Roberto, 00144 Roma (IT); Bilotti, Federico, 04011 Aorukua (Latina) (IT); Thompson, Brian James, Cincinnati Ohio 45226 (US)
(74) Representative: Leihkauf, Steffen Falk

(56) References cited:
- EP-A- 1 547 526
- WO-A-93/00868
- WO-A1-82/01644
- US-A- 4 567 891
- US-A1- 2002 022 852
- US-A1- 2005 228 442

## Description

The present invention relates, in general, to devices and methods for surgically modifying organs and vessels. More particularly, it relates to anastomosis devices for joining two organs such as, for example, two separate lengths of small bowel to each other, a section of small bowel to the stomach, or the common bile duct to the duodenum in a procedure called a choledochoduodenostomy. Vascular anastomosis may be performed as well.

The percentage of the world population suffering from morbid obesity is steadily increasing. Severely obese persons are susceptible to increased risk of heart disease, stroke, diabetes, pulmonary disease, and accidents. Because of the effect of morbid obesity to the life of the patient, methods of treating morbid obesity are being researched.

Numerous non-operative therapies for morbid obesity have been tried with virtually no permanent success. Dietary counseling, behavior modification, wiring a patient's jaws shut, and pharmacological methods have all been tried, and though temporarily effective, failed to correct the condition. Further, introducing an object in the stomach, such as an esophago-gastric balloon, to fill the stomach have also been used to treat the condition; however, such approaches tend to cause irritation to the stomach and are not effective long-term.

Surgical treatments of morbid obesity have been increasingly used with greater success. These approaches may be generalized as those that reduce the effective size of the stomach, limiting the amount of food intake, and those that create malabsorption of the food that is eaten. For instance, some patients benefit from adjustable gastric bands (AGB) that are laparoscopically placed about the stomach to form a stoma of a desired size that allows food to fill an upper portion of the stomach, causing a feeling of satiety. To allow adjustment of the size of the stoma after implantation, a fluid conduit communicates between an inwardly presented fluid bladder of the AGB to a fluid injection port subcutaneously placed in front of the patient's sternum. A syringe needle may then inject or withdraw fluid as desired to adjust the AGB.

Although an effective approach to obesity for some, other patients may find the lifestyle changes undesirable, necessitated by the restricted amount of food intake. In addition, the medical condition of the patient may suggest the need for a more permanent solution. To that end, surgical approaches have been used to alter the portions of the stomach and/or small intestine available for digesting food. Creating an anastomosis, or the surgical formation of a passage between two normally distinct vessels, is a critical step of many surgical procedures. This is particularly true of gastric bypass procedures in which two portions of small intestine are joined together and another portion of small intestine is joined to the stomach of the patient. This is also true of surgery to alleviate blockage in the common bile duct by draining bile from the duct to the small intestine during surgery for pancreatic cancer.

With particular reference to gastric bypass procedures, current methods of performing a laparoscopic anastomosis for a gastric bypass include stapling, suturing, and placing biofragmentable rings, each having significant challenges. For instance, suturing is time consuming, as well as being technique and dexterity dependent. Stapling requires placement of an anvil, which is a large device that cannot be introduced through a trocar port. Having to introduce the port through a laparotomy presents an increased incidence of wound site infection associated with intralumenal content being dragged to the laparotomy entry site.

For many anastomoses, surgeons use circular staplers, linear staplers, or manual sutures. However, to reduce incision size and to make the surgical process less technically demanding and time consuming, single and double piece anastomotic ring devices have been proposed which are deformed (in the case of a single piece device) or approximated (in the case of a double piece device) during endoscopic, laparoscopic or open surgery deployment by means of an apposite anastomotic applying instrumentation in order to hold tissue portions together. US 2005/0070926 A1 and US 2005/0070935 describe an anastomotic ring device with an unactuated shape of a cylinder with a proximal ring at one end and a distal ring at the other. The ring device further has proximal arms that are attached to the proximal ring and distal arms that are attached to the distal ring. Inwardly directed ends of the distal arms are coupled to inwardly directed ends of the proximal arms at a center ring such that the arms will outwardly actuate when the rings are drawn closer together during actuation of the applier. A latching mechanism comprises a hook protruding from the distal ring proximally such that it snap engages the proximal ring when the ring device assumes its actuated shape of a rivet. The anastomotic ring device disclosed in US 2005/0070926 A1 and US 2005/0070935 make it possible to perform a single lumen access anastomosis through existing trocar ports and to create an anastomotic attachment between lumens obviating the need for surgical stapling and suturing.

A double piece anastomotic ring device usually comprises two compression rings which are intended to be arranged on either side of the two tissue walls which need to be joined in anastomosis. The compression rings are approximated and locked to one another in order to clamp the two tissue walls between them. The anastomotic orifice through the tissue walls is then created by resecting the tissue overlapping into an internal passage opening of the compression rings. In this manner, the ring device stabilizes and maintains the shape of the anastomotic orifice.

Similarly to the above described single piece anastomotic device, the latching mechanism of the double piece anastomotic device comprises a hook or ratchet protruding from the first compression ring towards the second compression ring such that it engages a corresponding catch seat provided in the second compression ring when the rings are approximated to the desired degree of tissue compression.

While the known anastomotic devices are satisfactory from a technical point of view, the anastomoses created by means of the known devices are inevitably exposed to substances and mechanical stresses which can prejudice the desired healing process and the general post operative course of the patient. The exposure to substances is caused by the inevitable passage of fluids and contents (e.g. those passing through the digestive tract) which, apart from wearing the not yet completely healed tissue surrounding the anastomotic passage opening, can also carry corrosive or infective agents. The mechanical stresses acting on the anastomosis are mainly due to the passage of comparatively bulky surgical instrumentation through the anastomotic orifice.

WO-A-82/01644 discloses an anastomotic ring device according to the preamble of appended claim 1.

The object of the present invention is therefore to improve the known anastomotic ring devices such that the healing process of the resulting anastomoses is less affected by the inevitable passage of fluids, contents and surgical instrumentation through the anastomotic orifice.

This and other objects are achieved by an anastomosis device according to claim 1. Advantageous embodiments are the object of the dependent claims.

Thanks to its bushing portion, the locking device provides a lining which effectively covers and protects the inside surface of the anastomosis, thereby creating a channel for the passage of fluids, contents and surgical instruments which separates or isolates these agents from the surrounding tissue.

The provision of a distinct locking device separate from and connectable to both compression rings makes it possible to adjust the position of the proximal and distal compression rings and the tissue compression without obstruction by the locking device and to prevent undesired untimely locking of the ring device during positioning. Moreover, the distinct locking device can be utilized with a number of different anastomotic ring devices (of both the single piece type and double piece type) and makes it possible to transport the whole anastomotic device, for instance endoscopically, in single small parts to the anastomotic site.

In accordance with an important aspect of the invention, the bushing portion has a shape adapted to cover a prevalent portion of the inside surface of the clamped tissue portions, wherein the term "inside surface of the tissue portions" indicates the surface of the tissue clamped between the proximal and distal rings which faces radially inside the anastomotic passage opening. And the term "prevalent portion" means that the bushing portion covers more than 50% of the internal circumference of the anastomosis between the two rings.

According to a preferred embodiment, the bushing portion comprises a substantially tubular wall having a continuous or interrupted circular cross section.

Such a continuous or interrupted approximately circular cross-section of the bushing wall provides firstly an improved mechanical resistance against radial tissue pressure and, secondly, a maximized anastomotic passage aperture for a given central aperture of the compression rings and reduces the risk of leak between the two tissue layers.

According to an embodiment, the connectors of the locking device comprise elastically supported snapper teeth extending from the longitudinal portion radially outward to enable snap engagement of the distal compression ring. The elastic support of the snapper teeth can be advantageously obtained by forming the snapper teeth at longitudinally or circumferentially extending elastic beams which are preferably integral with the bushing wall. The elastic beams are advantageously arranged in window openings of the bushing wall in a manner that the window openings allow a radial movement of the elastic beams together with the snapper teeth and the elastic beams form a part of the bushing wall, thereby protecting the surrounding tissue. The gaps formed between the elastic beams and the bushing wall, as well as possible further interruptions of the bushing wall are preferably less than 30%, even more preferably less than 20% of the total bushing wall surface suitable to cover the inside surface of the clamped tissue portions.

According to a preferred embodiment, the bushing portion is sufficiently long to cover not only the internal surface of the clamped tissue portions, but also the internal surfaces of the proximal and distal compression rings, thereby providing a substantially continuous lining without interruptions or steps in the direction of flow through the anastomotic orifice.

According to a further embodiment, the snapper teeth or the elastic beams in general comprise a deviating surface, which is preferably inclined with respect to the direction of insertion of the locking device into the distal compression ring. The deviating surface is configured to slide, during insertion of the locking device, over a corresponding surface provided at the distal compression ring, thereby urging the snapper teeth elastically inward so that they can snap engage e.g. a circumferential edge of the distal compression ring by an elastically biased radially outward movement. This makes it possible to lock the compression rings to each other independently from their reciprocal angular position.

These and other features and advantages of the present invention shall be made apparent from the accompanying drawings which illustrate embodiments of the invention, and, together with the general description of the invention given above, and the detailed description of the embodiments given below, serve to explain the principles of the present invention.
- FIG. 1 is a perspective view of an anastomotic ring device for intralumenal anastomosis and a locking device according to a first embodiment of the invention.
- FIG. 2 is a perspective view of the ring device and the locking device of figure 1, wherein the locking device is inserted in the ring device and locks the latter in an actuated configuration.
- FIG. 3 is a proximal-lateral perspective view of a distal compression ring of the anastomotic ring device in figure 1.
- FIG. 4 is a proximal-lateral perspective view of a proximal compression ring of the anastomotic ring device in figure 1.
- FIG. 5 is a perspective, partially sectioned view of the proximal compression ring in figure 4.
- FIG. 6 is a distal-lateral perspective view of a needle group of the ring device in figure 1.
- FIG. 7 is a distal-lateral perspective view of a locking device according to an embodiment of the invention.
- FIG. 8 is a distal-lateral perspective view of a locking device according to a further embodiment of the invention.
- FIG. 9 is a distal-lateral perspective view of a locking device according to a yet further embodiment of the invention.
- FIG. 10 is a distal-lateral perspective view of a locking device according to a yet further embodiment of the invention.
- FIG. 11 is a distal-lateral perspective view of a locking device according to a yet further embodiment of the invention.
- FIG. 12 is a distal-lateral perspective view of a locking device according to a yet further embodiment of the invention.
- FIG. 13 is a proximal-lateral perspective view of a locking device according to a yet further embodiment of the invention.

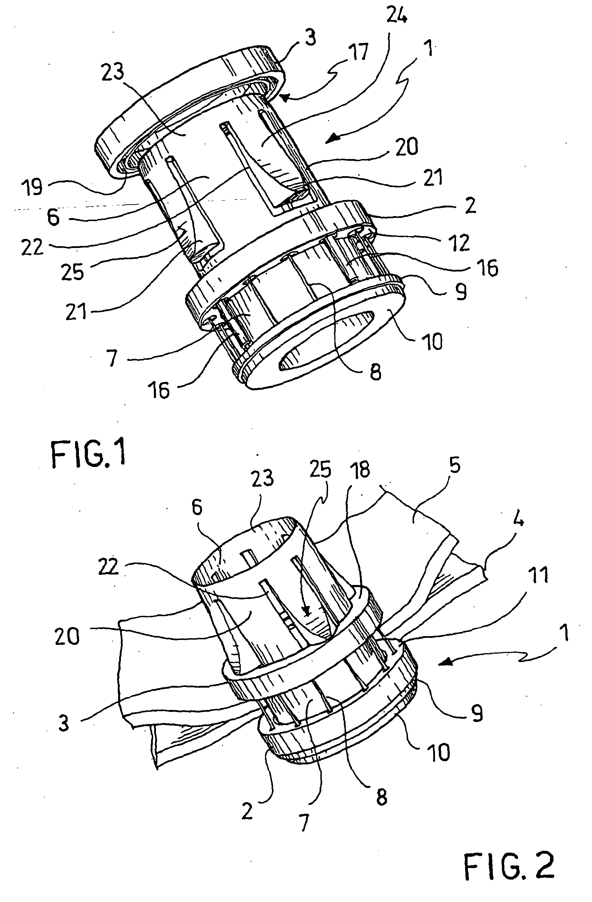

Turning to the drawings, wherein like numerals denote like components throughout the several views, FIG. 1 and 2 depict an anastomotic ring device 1.

The ring device 1 comprises at least a proximal compression ring 2 and a distal compression ring 3 intended to be positioned on either side of a proximal 4 and distal tissue portion 5 and suitable to clamp the proximal and distal tissue portions 4, 5 between them when the ring device 1 is actuated. Both compression rings 2, 3 define a central passage opening across the ring device 1. The ring device 1 further comprises a locking device 6 adapted to connect the distal 2 and proximal 3 rings in order to lock the ring device in its actuated configuration to form an anastomotic attachment between a distal tissue 5 (for instance intestinal wall tissue) and a proximal tissue 4 (for instance intestinal or gastric wall tissue) at an anastomosis target size, such as in a bariatric gastric bypass of a morbidly obese patient.

The locking device 6 is insertable in the passage opening of at least one of the proximal and distal rings 2, 3 and comprises a bushing portion 7 adapted to cover an inside surface of the tissue portions 4, 5 clamped between the compression rings 2, 3.

Preferably, the bushing portion 7 has a shape adapted to cover more than 50% of the internal circumference of the anastomosis between the two rings 2, 3. In this manner, the locking device 6 provides contemporaneously a locking function, a conservation of the shape of the anastomotic passage opening and a very efficient stenting feature.

According to a preferred embodiment, the bushing portion 7 comprises a substantially tubular wall having a continuous or interrupted approximately circular cross section.

Advantageously, the bushing portion 7 is sufficiently long to cover not only the internal surface of the clamped tissue portions 4, 5, but also the internal surfaces of the proximal and distal compression rings 2, 3, thereby providing a substantially continuous lining without interruptions or steps in the direction of flow through the anastomotic orifice.

Since the present invention concentrates mainly on the locking device 6 for the anastomotic device rather than on the anastomotic rings, figures 1 and 2 show an illustrative example of a double piece anastomotic ring device. It is not the intention of the inventors to limit the application of the locking device to the anastomotic device illustrated in figures 1 and 2. As a matter of fact, the locking device is also suitable to be used in connection with single piece anastomotic devices, as described and illustrated e.g. in US 2005/0070926 A1 and US 2005/0070935 and with other types of double piece anastomotic ring devices.

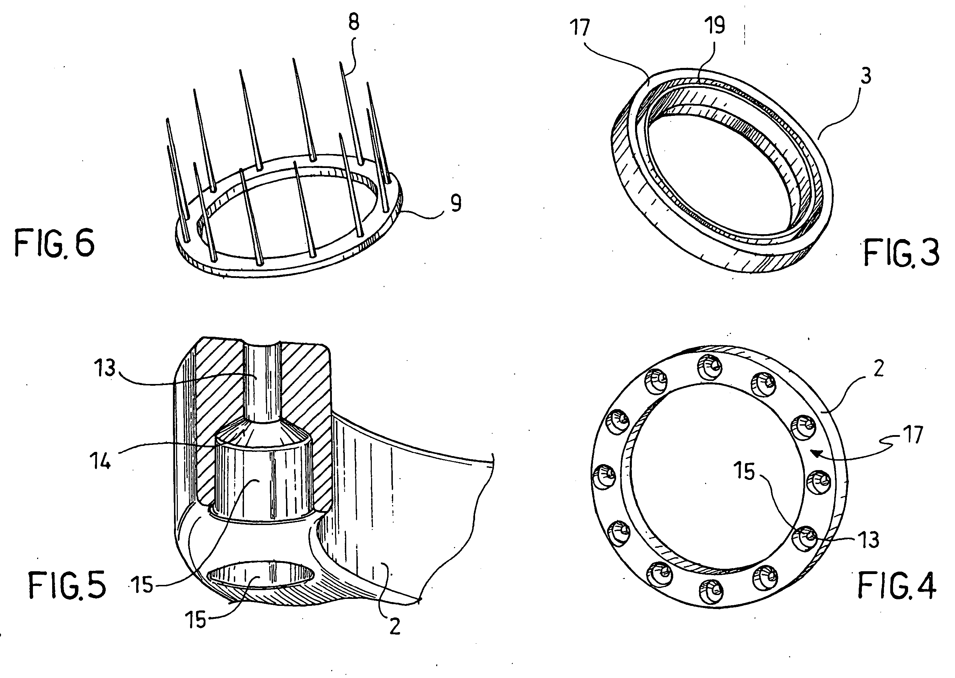

According to the embodiment illustrated in figures from 1 to 6, the locking device 6 defines a seat for receiving a group of needles 8 or staples such that, during a distal insertion movement of the locking device 6 into the passage openings of the distal and proximal rings 2, 3 the locking device pushes contemporaneously the group of needles 8 or staples in the anastomotic ring device piercing the tissue 3, 4 held between the proximal and distal compression rings. The needles 8 can be directly fixed to the locking device 6 or, as shown in figures 1 and 6, the group of needles 8 comprise a self supporting needle ring 9 which can be received by an apposite seat defined at the locking device 6. According to a preferred embodiment, the needle ring 9 is supported by a circumferential distal surface of a proximal annular shoulder 10 of the of the locking device 6.

The proximal compression ring 2 comprises a distal contact surface 11 intended to contact the proximal tissue portion 4 and an opposite proximal surface 12. The distal contact surface 11 is advantageously roughened in order to increase the friction between the proximal compression ring and the tissue necessary to prevent tissue slippage.

In case the anastomotic device comprises the needle group 8, the proximal compression ring 2 defines a plurality of axially extending through holes 13, each of which enabling guidance and penetration of a single needle 8 through the proximal compression ring 2. The alignment of the needles is further facilitated by providing a chamfered surface 14 around each through hole 13.

In accordance with a further embodiment, the proximal compression ring comprises proximally facing spring seats 15 adapted to receive compression springs or equivalent elastic compression means 16 arranged or clamped directly or indirectly between the annular shoulder 10 of the locking device 6 and the proximal compression ring 2 in order to bias the latter elastically towards the distal compression ring 3. The compression springs 16 guarantee a continuous compression force acting on the clamped tissue portions. Advantageously, the spring seats 15 are substantially coaxial with the through holes 13 and the chamfered surface 14 defines a proximally facing annular bottom surface of the spring seats 15. The elastic compression means 16 are e.g. embodied by tubular elastic o elastomeric elements inserted over the needles 8 or by helical compression springs.

The distal compression ring 3 comprises a proximal contact surface 17 intended to contact the distal tissue portion 5 and an opposite distal surface 18. The proximal contact surface 17 is advantageously roughened in order to increase the friction between the distal compression ring and the tissue necessary to prevent tissue slippage.

In case the anastomotic device comprises a needle group 8, the distal compression ring 3, particularly its proximal surface 17 further defines an annular needle receiving zone 19 adapted to be at least partially pierced or penetrated by the needle tips after the anastomotic device has been locked in its actuated configuration. In the embodiment shown in figures 1 and 3, the needle receiving zone 19 comprises a continuous circular groove or recess with two opposite locking surfaces suitable to prevent the needle tips from slipping radially out of engagement with the distal compression ring 3.

In the present description the terms "proximal" and "distal" are generally referred to the surgeons point of view and are used to enable an easy identification and understanding of the details of the anastomotic ring device and their mutual position. Nevertheless, such indication of position is not intended to limit the direction of application of the anastomotic ring device. As will be immediately appreciated by those skilled in the art, the direction of deployment of the anastomotic ring device and of the locking device might be also inverted without loosing the advantages thereof.

Turning now to the locking device 6, in accordance with a first embodiment illustrated in figures 1 and 2, the locking device 6 comprises an annular proximal shoulder 10 suitable to engage the proximal end surface 12 of the proximal ring 2 and a longitudinal portion 20 which protrudes distally from the proximal shoulder 10 and forms the bushing portion 7 and one or more, preferably four, elastically supported snapper teeth 21 adapted to snap engage the distal surface 18 of the distal compression ring 12. In particular, the bushing portion 7 is formed by a proximal part of the longitudinal portion adjacent to the proximal shoulder 10. The bushing portion 7 has a continuous circular cylindrical wall adapted to cover and protect the tissue clamped between the compression rings and to keep the anastomotic orifice open (stenting action). Distally from the bushing portion, the cylindrical wall defines longitudinal window openings 22 which are distally delimited by a continuous circular end ring 23. Elastic cantilever beams 24 protrude from the end ring 23 proximally into the window openings 22 and provide an elastic support for the snapper teeth 21 which are preferably formed at or near a proximal end of the elastic beams 24 and which extend radially outward.

In order to facilitate the snap engagement between the locking device and the distal compression ring, the elastic beams 24 comprise a sloped deviating surface 25 configured to slide, during insertion of the locking device, over an internal edge of the distal compression ring, thereby urging the elastic beams 24 elastically inward so that the snapper teeth 21 automatically snap engage the distal surface of the distal compression ring by an elastically biased radially outward movement.

The end ring 23 or, more generally, the distal end of the longitudinal portion 20 of the locking device 6 is preferably distally tapered to facilitate the insertion of the locking device across the tissue layers and/or into the ring device.

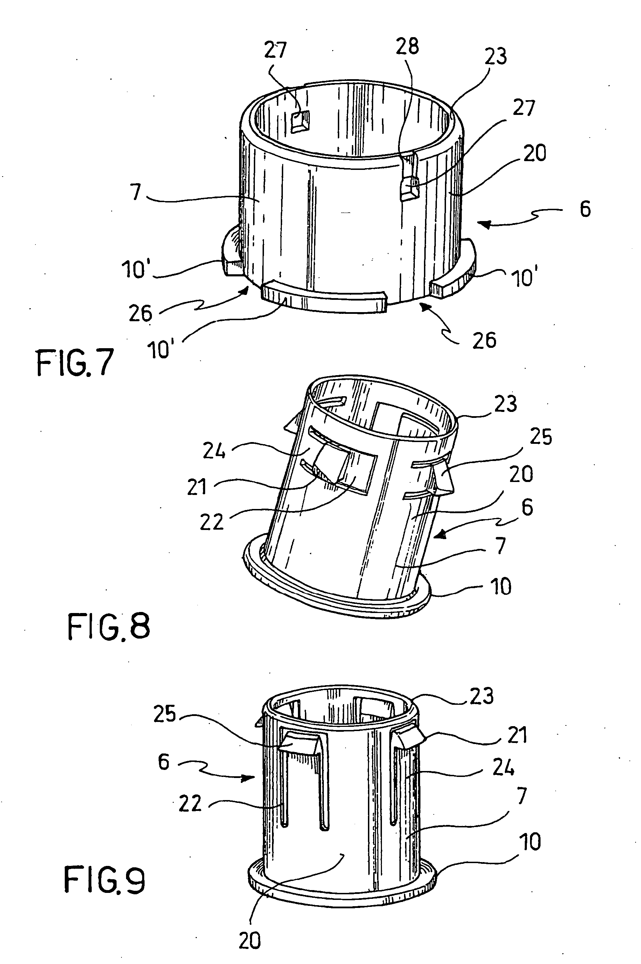

Figure 7 illustrates a second embodiment of the locking device 6, wherein the proximal shoulder is embodied by a number of distinct radially protruding shoulder segments 10' circumferentially spaced to one another. The gaps 26 between adjacent shoulder segments 10' result in a weight reduction and provide the space for the passage of components of anastomotic ring appliers which might need to be inserted and withdrawn from the ring device and which would otherwise interfere with the proximal shoulder of the locking device. As will be described later on, the gaps 26 can also provide engagement seats for corresponding portions of a ring applier adapted to rotate the locking device during application.

While the interrupted proximal shoulder of the locking device according to the second embodiment is compatible and, if desired, interchangeable with the proximal shoulder of the first embodiment, the connecting means of the second embodiment (fig. 7) differs substantially from that of the first embodiment (fig. 1). According to the second embodiment, the longitudinal portion 10 comprises a substantially cylindrical wall which is, at least in its distal end region, radially elastically deformable to an extent to allow two oppositely arranged pins which protrude radially inward from the distal compression ring (not shown in the figure) to snap into corresponding catches or recesses 27 formed in the cylindrical wall. Advantageously, the end ring 23 of the longitudinal portion 10 comprises external longitudinal grooves 28 which extend from its distal edge towards and into the recesses 27. The grooves 28, which doesn't go through the entire radial thickness of the end ring 23, facilitate the mutual positioning and snap engagement of the pins and recesses 27. In this case, the elastic snap effect of the locking device is obtained by the elastic deformability of the distal end region of the longitudinal portion 10.

Figure 8 illustrates a third embodiment of the locking device 6, which is generally very similar to the first embodiment (fig. 1) apart from the window openings 22 and the elastic cantilever beams 24 which extend in a circumferential direction, thereby reducing the longitudinal extension of the window openings 22. This results in a reduction of the longitudinal dimension of the locking device and makes it possible to form the bushing portion 7 as an uninterrupted continuous tubular wall suitable to cover completely the inside surfaces of the tissue portions 4, 5. With the elastic beam 24 extending circumferentially, the deviating surfaces 25 are preferably formed on a distal face of the snapper teeth 21.

Figure 9 illustrates a fourth embodiment of the locking device 6, wherein the longitudinal portion 20 comprises a substantially cylindrical wall defining longitudinal window openings or slots 22 and the snapper teeth 21 are formed on radially elastically deflectable beams 24 arranged inside the window openings or slots 22 (and e.g. cantilevered on the proximal end ring of the locking device).

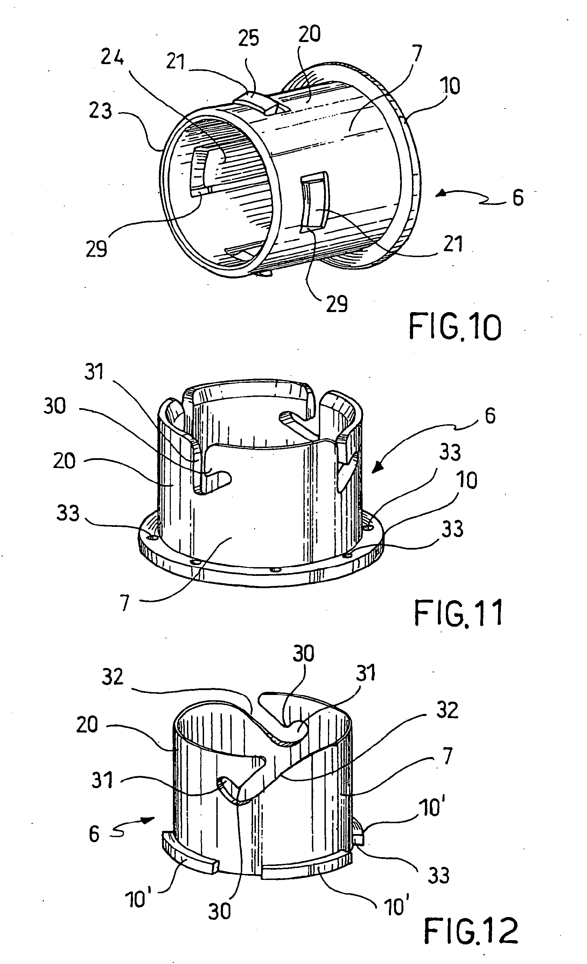

Figure 10 illustrates a fifth embodiment of the locking device 6, very similar to the fourth embodiment (fig. 9), wherein the elastic beams 24 are arranged radially inside the tubular wall of the longitudinal portion 20 and only the snapper teeth 21 protrude radially outward through corresponding window openings 29. In this way, the elastic beams 24 are protected by the tubular wall which prevents the clamped tissue 4, 5 from urging the beams inward and accidentally releasing the anastomotic ring device. As will be readily appreciated by those skilled in the art, such a wall protecting the elastic beams 24 can be advantageously implemented also in the other embodiments, particularly in that illustrated in Fig. 1.

Figure 11 illustrates a sixth embodiment of the locking device 6, wherein the tubular wall of the longitudinal portion 21 forms a plurality of, preferably four, hooks 30 which are arranged at constant circumferential intervals. The hooks 30 are advantageously formed by grooves 31 in the tubular wall which are curved or angled such that they define an undercut with respect to the longitudinal direction of the locking device adapted to retain corresponding pins formed at the distal compression ring. While this embodiment has the advantage that the hook feature doesn't protrude out of the normal thickness of the tubular wall and that no elastic deformation is required for locking the anastomotic ring device, it requires a relative rotational movement between the distal ring and the locking device in order to perform the connection.

Figure 12 shows a further development of the embodiment in figure 11, wherein the grooves 31, particularly their segments 32 which extend from the distal edge of the tubular wall towards the hooks 30, are sloped with respect to the longitudinal direction in a manner that during insertion of the locking device into the distal ring, the pins slide along the sloped portions 32 of the grooves 31, thereby causing the relative rotation between the locking device and the distal compression ring.

The locking devices shown in figures 11 and 12 may advantageously comprise spanner surfaces 33 adapted to be engaged by a spanner device of an applier (not shown) to facilitate the rotation of the locking device 6 during deployment. According to an illustrative embodiment (fig. 11) the spanner surfaces 33 are embodied by a plurality of holes in the proximal shoulder 10. Alternatively, the spanner surfaces 33 can be embodies by radial end surfaces of the shoulder segments 10' (fig. 12).

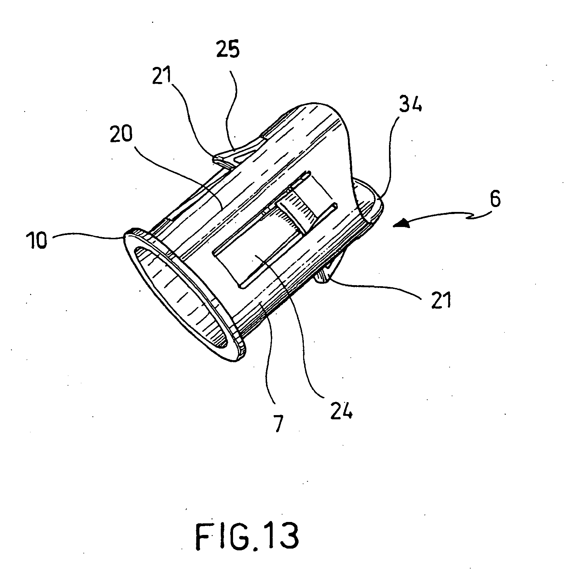

Figure 13 illustrates an eighth embodiment of the locking device 6, wherein the distal end of the longitudinal portion 20, i.e. the distal end 34 of its tubular wall, is inclined with respect to a plane perpendicular to the longitudinal extension of the locking device, thereby facilitating the insertion of the longitudinal portion across the tissue layers 4, 5 and the compression rings 2, 3. Advantageously, the distal end 34 of the tubular wall is wavy and preferably but not necessarily fish mouth shaped.

According to the embodiment illustrated in figure 13, the elastic beams 24 are not cantilevered but supported on both ends by the tubular wall so that they have an increased bending stiffness and a more stable positioning during insertion of the locking device.

Preferred materials for the fabrication of the anastomotic ring device and its single components are biocompatible and advantageously bio-fragmentable or bio-absorbable materials, e.g. polymers.

Even though a number of features of the anastomotic ring device and of the locking device according to the present invention have been described and illustrated by means of single distinct embodiments, it readily appears to those skilled in the art that most of the described features are perfectly compatible to one another and can be therefore combined in a single device. The very rare exceptions and cases of incompatibility between features of different illustrative embodiments have been expressly indicated in the foregoing description.

While the present invention has been illustrated by description of several embodiments and while the illustrative embodiments have been described in considerable detail, it is not the intention of the applicant to restrict or in any way limit the scope of the appended claims to such detail. Additional advantages and modifications may readily appear to those skilled in the art.

For example, aspects of the invention have application to surgical procedures performed endoscopically and laparoscopically, as well as an open procedure. Use herein of one of these or similar terms should not be construed to limit the present invention for use in only one category of surgical procedure.

For another example, even though a procedure has been described wherein the locking device is introduced in the compression rings after the positioning of the latter at the anastomotic site, it is also possible and advantageous to firstly insert the locking device with its bushing portion in the hole formed in the tissue portions and successively insert the compression ring or rings over the locking device.

Moreover, as has already be evidenced during the description of the single embodiments, the bushing portion can be rigid or (e.g. elastically) pliable.

For another example, although bariatric procedures for bypassing portions of a gastrointestinal tract are referred to, it should be appreciated that other surgical procedures may benefit by a locking device and an anastomotic ring device having aspects described herein, such as for gastro-jejunostomy, jejuno-jejunostomy, colo-proctostomy, jejuno-colostomy or anastomoses involving the Chole duct, the bile duct and vascular bypasses.

## Claims

1. An anastomosis device comprising;
- a ring device (1) heaving a proximal ring (2) and a distal ring (3) intended to be positioned on either side of a proximal (4) and distal tissue portion (5) and suitable to clamp the proximal and distal tissue portions (4, 5) between them when the ring device (1) is actuated, wherein both rings (2, 3) define a central passage opening across the ring device (1),
- a locking device (6) adapted to connect the distal and proximal rings (2, 3) to lock the ring device (1) in its actuated configuration,
wherein said locking device (6) is distinct from both the proximal ring (2) and the distal ring (3); **characterised in that** said locking device comprises:
- a proximal shoulder (10) engaging a proximal end surface (12) of said proximal ring (2),
- a longitudinal portion (20) distally protruding from the proximal shoulder (10) and inserted in the passage openings of both the proximal and distal rings (2, 3), said longitudinal portion (20) forming a bushing portion (7) adapted to cover an inside surface of the tissue portions (4, 5) when they are clamped between said proximal and distal rings (2, 3) and one or more connectors (21; 30) adapted to engage the distal compression ring (3).

2. An anastomosis device according to claim 1, wherein the bushing portion (7) comprises a substantially tubular wall suitable to cover a prevalent portion of the inside surface of the clamped tissue portions (4, 5).

3. An anastomosis device according to claim 2, wherein said bushing portion (7) has a circumferentially continuous, uninterrupted cross-section.

4. An anastomosis device according to any one of claims 1 - 3, wherein said connectors (21; 30) comprise elastically supported snapper teeth (21) adapted to snap engage the distal compression ring (3).

5. An anastomosis device according to the preceding claim, wherein the said snapper teeth (21) are elastically supported by elastic beams (24) integrally formed with the tubular wall of the bushing portion (7).

6. An anastomosis device according to any one of claims 1 - 3, wherein said connectors (21; 30) comprise hooks (30) formed by grooves (31) in the tubular wall of the longitudinal portion (20), said grooves (31) being adapted to receive and guide corresponding pins of the distal compression ring (3) and curved such that they define an undercut with respect to the longitudinal direction of the locking device (6) adapted to retain said pins.

7. An anastomosis device according to the preceding claim, wherein said grooves (31) comprise segments (32) which extend from a distal edge of the tubular wall towards the hooks (30) and which are sloped with respect to the longitudinal extension of the locking device (6) in a manner to cause a relative rotation between the distal compression ring (3) and the locking device (6) in response to their approximation.

8. An anastomosis device according to any one of the preceding claims, wherein a distal end of the tubular wall of said longitudinal portion (20) is inclined with respect to a plane perpendicular to the longitudinal extension of the locking device (6).

9. An anastomosis device according to the preceding claim, wherein the distal end of said tubular wall is circumferentially wavy and preferably, fish mouth shaped.

10. An anastomosis device according to the preceding claims, comprising a group of needles (8) which can be applied to the compression rings and pierced through the tissue (3, 4) held between the compression rings.

## Patentansprüche

1. Anastomosevorrichtung, umfassend:
eine Ringvorrichtung (1), die einen proximalen Ring (2) und einen distalen Ring (3) aufweist, die bestimmungsgemäß auf jeder Seite eines proximalen (4) und distalen Gewebeabschnitts (5) anzuordnen sind und geeignet sind, die proximalen und distalen Gewebeabschnitte (4, 5) zwischen sich zu klemmen, wenn die Ringvorrichtung (1) betätigt wird, wobei beide Ringe (2, 3) eine zentrale Durchlassöffnung durch die Ringvorrichtung (1) definieren,
eine Verriegelungsvorrichtung (6), die so ausgeführt ist, dass sie die distalen und proximalen Ringe (2, 3) verbindet, um die Ringvorrichtung (1) in ihrer betätigten Konfiguration zu verriegeln, wobei die Verriegelungsvorrichtung (6) sowohl vom proximalen Ring (2) als auch vom distalen Ring (3) verschieden ist;
**dadurch gekennzeichnet, dass** die Verriegelungsvorrichtung umfasst:
eine proximale Schulter (10), die mit einer proximalen Endfläche (12) des proximalen Rings (2) in Eingriff gelangt,
einen longitudinalen Abschnitt (20), der von der proximalen Schulter (10) distal hervorsteht und in die Durchlassöffnungen sowohl des proximalen als auch des distalen Rings (2, 3) eingesetzt ist, wobei der longitudinale Abschnitt (20) einen Buchsenabschnitt (7) bildet, der so ausgeführt ist, dass er eine Innenfläche der Gewebeabschnitte (4, 5) abdeckt, wenn diese zwischen dem proximalen und dem distalen Ring (2, 3) geklemmt sind und einem oder mehreren Verbindern (21; 30), die so ausgeführt sind, dass sie mit dem distalen Kompressionsring (3) in Eingriff gelangen.

2. Anastomosevorrichtung nach Anspruch 1, wobei der Buchsenabschnitt (7) eine im Wesentlichen rohrförmige Wand umfasst, die geeignet ist, einen überwiegenden Anteil der Innenfläche der geklemmten Gewebeabschnitte (4, 5) abzudecken.

3. Anastomosevorrichtung nach Anspruch 2, wobei der Buchsenabschnitt (7) einen im Umfangsrichtung kontinuierlichen, ununterbrochenen Querschnitt aufweist.

4. Anastomosevorrichtung nach irgendeinem der Ansprüche 1 bis 3, wobei die Verbinder (21; 30) elastisch unterstützte Schnappzähne (21) umfassen, die so ausgeführt sind, dass sie am distalen Kompressionsring (3) einschnappen.

5. Anastomosevorrichtung nach dem vorangehenden Anspruch, wobei die Schnappzähne (21) durch elastische Ausleger (24) elastisch unterstützt sind, die integral mit der rohrförmigen Wand des Buchsenabschnitts (7) ausgebildet sind.

6. Anastomosevorrichtung nach irgendeinem der Ansprüche 1 bis 3, wobei die Verbinder (21; 30) Haken (30) umfassen, die durch Nuten (31) in der rohrförmigen Wand des longitudinalen Abschnitts (20) ausgebildet werden, wobei die Nuten (31) so ausgeführt sind, dass sie entsprechende Stifte des distalen Kompressionsrings (3) aufnehmen und führen, und so gekrümmt sind, dass sie einen Unterschnitt bezüglich der Longitudinalrichtung der Verriegelungsvorrichtung (6) definieren, der so ausgeführt ist, dass er die Stifte zurückhält.

7. Anastomosevorrichtung nach dem vorangehenden Anspruch, wobei die Nuten (31) Segmente (32) umfassen, die sich von einem distalen Rand der rohrförmigen Wand in Richtung zu den Haken (30) erstrecken, und die bezüglich der longitudinalen Ausdehnung der Verriegelungsvorrichtung (6) abgeschrägt sind, derart, dass sie eine relative Rotation zwischen dem distalen Kompressionsring (3) und der Verriegelungsvorrichtung (6) in Reaktion auf deren Annäherung hervorrufen.

8. Anastomosevorrichtung nach irgendeinem der vorangehenden Ansprüche, wobei ein distales Ende der rohrförmigen Wand des longitudinalen Abschnitts (20) bezüglich einer Ebene senkrecht zur longitudinalen Ausdehnung der Verriegelungsvorrichtung (6) geneigt ist.

9. Anastomosevorrichtung nach dem vorangehenden Anspruch, wobei das distale Ende der rohrförmigen Wand in Umfangsrichtung wellig und vorzugsweise fischmaulförmig ist.

10. Anastomosevorrichtung nach irgendeinem der vorangehenden Ansprüche, die eine Gruppe von Nadeln (8) umfasst, die an den Kompressionsringen angebracht und durch das zwischen den Kompressionsringen gehaltene Gewebe (3, 4) gestochen werden können.

## Revendications

1. Dispositif d'anastomose comprenant :
- un dispositif à anneaux (1) ayant un anneau proximal (2) et un anneau distal (3) destinés à être positionnés de chaque côté d'une partie de tissu proximale (4) et d'une partie de tissu distale (5) et appropriés pour clamper les parties de tissu proximale et distale (4, 5) entre eux quand le dispositif à anneaux (1) est actionné, où les deux anneaux (2, 3) définissent une ouverture de passage centrale dans le dispositif à anneaux (1),
- un dispositif de blocage (6) adapté pour relier les anneaux distal et proximal (2, 3) pour bloquer le dispositif à anneaux (1) dans sa configuration actionnée,
où ledit dispositif de blocage (6) est distinct de l'anneau proximal (2) et de l'anneau distal (3) ; **caractérisé en ce que** ledit dispositif de blocage comprend :
- un épaulement proximal (10) coopérant avec une surface terminale proximale (12) dudit anneau proximal (2),
- une partie longitudinale (20) faisant saillie distalement de l'épaulement proximal (10) et insérée dans les ouvertures de passage des anneaux proximal et distal (2, 3), ladite partie longitudinale (20) formant une partie formant douille (7) adaptée pour couvrir une surface interne des parties de tissu (4, 5) quand elles sont clampées entre lesdits anneaux proximal et distal (2, 3) et un ou plusieurs connecteurs (21 ; 30) adaptés pour coopérer avec l'anneau de compression distal (3).

2. Dispositif d'anastomose selon la revendication 1 où la partie formant douille (7) comprend une paroi sensiblement tubulaire appropriée pour couvrir une partie prédominante de la surface interne des parties de tissu clampées (4, 5).

3. Dispositif d'anastomose selon la revendication 2 où ladite partie formant douille (7) a une section transversale ininterrompue, continue sur la circonférence.

4. Dispositif d'anastomose selon l'une quelconque des revendications 1-3 où lesdits connecteurs (21 ; 30) comprennent des dents d'encliquetage (21) supportées élastiquement adaptées pour coopérer par encliquetage avec l'anneau de compression distal (3).

5. Dispositif d'anastomose selon la revendication précédente où lesdites dents d'encliquetage (21) sont supportées élastiquement par des nervures élastiques (24) formées d'une pièce avec la paroi tubulaire de la partie formant douille (7).

6. Dispositif d'anastomose selon l'une quelconque des revendications 1-3 où lesdits connecteurs (21 ; 30) comprennent des crochets (30) formées par des rainures (31) dans la paroi tubulaire de la partie longitudinale (20), lesdites rainures (31) étant adaptées pour recevoir et guider des ergots correspondants de l'anneau de compression distal (3) et incurvées de sorte qu'elles définissent un évidement par rapport à la direction longitudinale du dispositif de blocage (6) adapté pour retenir lesdits ergots.

7. Dispositif d'anastomose selon la revendication précédente où lesdites rainures (31) comprennent des segments (32) qui s'étendent depuis un bord distal de la paroi tubulaire vers les crochets (30) et qui sont inclinés par rapport à l'extension longitudinale du dispositif de blocage (6) de manière à provoquer une rotation relative entre l'anneau de compression distal (3) et le dispositif de blocage (6) en réponse à leur approche.

8. Dispositif d'anastomose selon l'une quelconque des revendications précédentes où une extrémité distale de la paroi tubulaire de ladite partie longitudinale (20) est inclinée par rapport à un plan perpendiculaire à l'extension longitudinale du dispositif de blocage (6).

9. Dispositif d'anastomose selon la revendication précédente où l'extrémité distale de ladite paroi tubulaire est ondulée suivant la circonférence et, de préférence, en forme de bouche de poisson.

10. Dispositif d'anastomose selon les revendications précédentes comprenant un groupe d'aiguilles (8) qui peuvent être appliquées aux anneaux de compression et qui peuvent percer le tissu (3, 4) maintenu entre les anneaux de compression.
